# EUROPEAN PATENT SPECIFICATION

(11) **EP 0 586 516 B1**
(45) Date of publication and mention of the grant of the patent: **11.09.1996**
(21) Application number: 92912086.3
(22) Date of filing: 28.05.1992
(51) Int. Cl.: A61B 17/02, A61B 19/00

(54) **PROPERITONEAL MECHANICAL RETRACTION APPARATUS**
MECHANISCHE RETRAKTOR-VORRICHTUNG FÜR DAS PERITONEUM
ECARTEUR MECANIQUE PROPERITONEAL

(30) Priority: 29.05.1991 US 706781
(43) Date of publication of application: 16.03.1994
(73) Proprietor: ORIGIN MEDSYSTEMS, INC., Menlo Park, CA 94025 (US)
(72) Inventor: MOLL, Frederic, H., San Francisco, CA 94118 (US); CHIN, Albert, K., Palo Alto, CA 94303 (US); KAUFMANN, Rick, J., Los Gatos, CA 95030 (US); GRESL, Charles, Jr., San Francisco, CA 94114 (US)
(74) Representative: Schmitz, Jean-Marie
(86) International application number: PCT/US92/04456
(87) International publication number: WO 92/21294

(56) References cited:
- WO-A-91/02493
- WO-A-91/14392
- US-A- 2 841 148
- US-A- 4 052 980
- US-A- 4 705 040
- Surgical Laparoscopy and Endoscopy, vol. 1, no. 2, 27 June 1991, Raven Press Ltd, (New York, US), M.M. GAZAYERLI: "The gazayerli endoscopic retractor Model 1", pages 98-100, see whole document (cited in the application)

## Description

The present invention concerns an apparatus for lifting an abdominal wall.

The invention relates to devices for use in laparoscopic surgery, in particular, to devices that lift the abdominal wall to provide working space in which to carry out laparoscopic procedures.

Laparoscopy dates back to the turn of the 20th Century. Early laparoscopic techniques were used primarily for diagnostic purposes to view the internal organs, without the necessity of conventional surgery. Since the 1930s, laparoscopy has been used for sterilization and, more recently, for suturing hernias. U.S. Patents 4,919,152 and 4,944,443 are concerned with techniques for suturing hernias. Another recent innovation is the use of laparoscopic surgery for removing the gallbladder.

In the course of performing laparoscopic procedures in the abdomen, it is necessary to raise the abdominal wall to create space in which to work. A well-known method of raising the abdominal wall is to insufflate the abdominal cavity with a suitable insufflation gas, such as air, or carbon dioxide. A significant disadvantage of gas insufflation is that instruments must be passed into the abdominal cavity through gas-tight seals, which significantly reduce the surgeon's feel of the instruments.

Several mechanical alternatives to gas insufflation have been proposed. The Gazayerli Endoscopic Retractor Model 1, described in SURGICAL LAPAROSCOPY AND ENDOSCOPY, Vol. 1, No. 2, 1991, pages 98-100, published after the priority date of the present invention, has a rigid rod with a hinged blade at the distal end. The blade can rotate through 360 degrees about an axis perpendicular to the long axis of the rod. The blade is aligned with the long axis of the rod for insertion into the abdomen through a small puncture. Once inside the abdomen, the blade is swivelled through about 90 degrees to form a T-shaped structure. The proximal end of the rod can be raised by hand or by a rope, pulley and weight arrangement. Raising the rod causes the blade to engage the abdominal wall and to lift it.

Published International Application under the Patent Cooperation Treaty No. WO 91/14392, which has entered the regional phase and is thus relevant under Art. 54(3) EPC, shows a wire structure that is threaded into the belly through a small puncture to engage and to lift the abdominal wall. The application also shows a fan retractor that has a first angle-shaped member hang a tubular first leg having a bore, a second leg that engages with the abdominal wall, and a third leg, remote from the second leg, that has a hook-shaped member on its end distal from the first leg. The second leg is depicted as having a rectangular cross section that remains constant along its length and is considerably thicker than it is wide. A second angle-shaped member has a first leg that pivots within the bore of the first leg of the first angle-shaped member, a second leg that engages with the abdominal wall, and a third leg, remote from the second leg, that serves as a operating lever for the second angle-shaped member. The second leg is also depicted as having a rectangular cross section that remains constant along its length and is considerably thicker than it is wide.

The second legs of the angle-shaped members are closed together to insert them into the abdominal cavity through an incision. The third leg of the second angle-shaped member is then operated to spread the second leg of the second angle-shaped member apart from the second leg of the first angle-shaped member. The first legs are engaged with the peritoneum inside the abdominal cavity. A lifting force is then applied to the hook-shaped member to lift the retractor and hence to lift the abdominal wall.

The known fan retractors are all intended for intra-abdominal placement. It is difficult to place the peritoneum-engaging elements of such devices inside the abdominal cavity adjacent to the peritoneum without snagging the bowel or omentum. It is often necessary to make multiple attempts at inserting the retractor before the fan retractor can be correctly positioned with its peritoneum-engaging elements adjacent to the peritoneum with no bowel or omentum caught between the peritoneum-engaging elements and the peritoneum. Insufflating the abdomen before inserting the fan retractor does not eliminate the risk of snagging.

If the retractor is inserted, lifted, and maintained in a lifted state with an unrecognized loop of bowel caught between the peritoneum-engaging elements of the retractor and the peritoneum, trauma or necrosis to that loop of bowel may occur, with significant morbidity or mortality.

Known fan retractors have a substantially constant stiffness along the length of their peritoneum-engaging elements. This causes the pressure that the peritoneum-engaging elements exert against the peritoneum to increase sharply towards the ends of the peritoneum-engaging elements. High pressure can cause trauma to the peritoneum, and there is a risk that the end of the peritoneum-engaging elements will penetrate the peritoneum.

The peritoneum-engaging elements of known fan retractors move independently of one another. This can lead to the peritoneum-engaging elements of the fan retractor being asymmetrically placed within the abdominal cavity, which results in the peritoneum-engaging elements providing the retracting force unequally. With asymmetrical placement, there is the risk that the more heavily loaded peritoneum engaging element will traumatize or penetrate the peritoneum.

The lifting force applied by known fan retractors is generally determined by the lifting result obtained. If, for some reason the abdominal wall fails to lift, the lifting force could accidentally be increased to the point at which trauma to or penetration of the peritoneum occurs.

Known fan retractors are rigidly attached to lifting bars such that, if the lifting bar is carelessly lowered at the end of treatment, the lifting bar can push the fan retractor into the abdomen, and cause a compression injury to the underlying organs. The apparatus for lifting an abdominal wall of the present invention is defined in claim 1.

In the following description, the word "organ" will be used to mean an organ or a tissue that is retracted by a retraction device. The word "treat" will be used to mean both treat and observe, and the word "treatment" will be used to mean both treatment and observation. The word "tissue* or the phrase "tissue to be treated" will both be used to mean the organ or the tissue that is treated through or inside a retraction device.

A fan retractor according to the invention has a pair of angle-shaped elements moveable relative to one another. The angle-shaped elements include first legs disposed in a generally parallel relationship, and second legs that extend laterally from the first legs, and are adapted to engage with the abdominal wall. The second legs fan out upon rotation of the first legs relative to one another. Finally, the retractor includes a lifting device that applies a lifting force directly to the first leg of each angle-shaped element.

In a version of the fan retractor according to the invention for properitoneal use, that is, with the second legs of the retractor inserted between the peritoneal fat layer and the peritoneum, the second legs have a thickness that decreases with increasing distance from the first legs. With this placement of the retractor, the peritoneum provides a drape over the bowel and omentum and prevents the second legs of the retractor from snagging the bowel or the omentum.

Providing the second legs with a thickness that decreases distally from the first legs enables the second legs act as a wedge to separate the peritoneum from the properitoneal fat layer. The stiffness of such second legs decreases distally from the first legs, which enables the second legs to flex, and to conform to the curvature of the abdominal wall. The second legs conforming to the curvature of the abdominal wall enables the second legs to exert a lifting force to the abdominal wall that is substantially constant along the length of the second legs. This reduces the risk of the distal ends of the second legs traumatizing or penetrating the abdominal wall. This advantage is obtained both when the fan retractor is used properitoneally and when the fan retractor is used normally, i.e., when the fan retractor is used to apply the lifting force to the posterior side of the peritoneum.

In a further variation, the fan retractor according to the invention additionally includes a contrarotating device that responds to rotation of the first leg of one of the angle-shaped elements through a first angle. The contrarotating device rotates the first leg of the other of the first angle-shaped elements through an angle substantially equal and opposite to the first angle. The contrarotating device causes the second legs to fan out symmetrically. This feature is especially useful when the fan retractor is used properitoneally, since the position of the second legs cannot be directly observed when the second legs are hidden by the peritoneum. With symmetrical fanning, the risk that one of the second legs may inadvertently abut, and possibly pierce, the abdominal side wall id significantly reduced.

In a yet further variation, the fan retractor according to the invention additionally includes a lifting force measuring device that is coupled to the lifting device and that measures the lifting force. This enables the lifting force to be monitored during the lifting operation to prevent an excessive lifting force, that could possibly result in trauma to or penetration of the abdominal wall, from being applied to the abdominal wall.

In a further variation, the lifting device of a fan retractor according to the invention additionally includes a unidirectional coupler that applies the lifting force to the angle-shaped elements in the lifting direction, and applies a substantially zero force to the angle-shaped elements in a direction opposite to the lifting direction. This reduces the risk of compression injury to the bowel or omentum occurring as a result of a force in the direction opposite to the lifting direction imposed on the coupling. This can occur if the lifting bar to which the coupling is attached is lowered too far.

Finally, at least one of the second legs of a fan retractor according to the invention may additionally include an internal passage. The internal passage may be used to provide aspiration and infusion in its vicinity. Additionally or alternatively, the internal passage may carry an optical fibre through which light to illuminate the treatment site can fed from an external light source.

In a method of properitoneally lifting the abdominal wall, a fan retractor according to the invention is provided. An incision is made through the abdominal wall to the properitoneal fat layer. The distal ends of the second legs are introduced through the incision to separate the peritoneum from the properitoneal fat layer. The second legs are advanced under the peritoneum. The first legs are rotated relative to one another to fan out the second legs, and a lifting force is applied directly to the first leg of each angle-shaped element via lifting means.

In another method using the apparatus according to the invention an incision is made through the abdominal wall, and the distal ends of the second legs are advanced through the invention. The first legs are rotated relative to one another to fan out the second legs, and the second legs are engaged with the abdominal wall. Finally, a lifting force is applied as in the first method.

In yet another method using a preferred embodiment of the apparatus according to the invention an incision through the abdominal wall, and the second legs are advanced into the incision. The first legs are rotated relative to one another to fan out the second legs, and the second legs are engaged with the abdominal wall. A lifting force is applied to the angle-shaped elements like in the previously mentioned methods, and the lifting force is observed using the force measuring means.

In order that the invention is fully understood reference is made on the accompanying drawings, wherein:
Figure 1 is a perspective view of a simplified version of a fan retractor according to the invention.
Figure 2A is a cross sectional view of the leg actuators of a fan retractor according to the invention, taken along the line X-X' in figure 1.
Figure 2B is a perspective view of the leg actuators of a fan retractor according to the invention.
Figure 3A is a perspective view of the preferred embodiment of the second legs of a fan retractor according to the invention in their closed position.
Figure 3B is a cross sectional view of the first part of the second legs of a fan retractor according to the invention, taken on the line A-A' in figure 3A.
Figure 3C is a cross sectional view of the second part of the second legs of a fan retractor according to the invention, taken on the line B-B' in figure 3A.
Figure 3D is a cross sectional view of the third part of the second legs of a fan retractor according to the invention, taken on the line C-C' in figure 3A.
Figure 3E is a cross sectional view of the fourth part of the second legs of a fan retractor according to the invention, taken on the line D-D' in figure 3A.
Figure 3F is a cross sectional view of the first part of an alternative embodiment of the second legs of a fan retractor according to the invention, in which the first part of the second legs has an oval cross-section.
Figure 3G is a cross sectional view of the second part of an alternative embodiment of the second legs of a fan retractor according to the invention, in which the first part of the second legs has an oval cross-section.
Figure 4A is a perspective view of the mounting block and lifting bar adaptor of a simplified version of the fan retractor according to the invention.
Figure 4B is a cross sectional view of the mounting block and lifting bar adaptor of a simplified version of the fan retractor according to the invention, taken along the line Y-Y'.
Figure 5A is a perspective view of the preferred embodiment of a fan retractor according to the invention.
Figure 5B is a plan view of a first embodiment of the additional lifting force indicator of a fan retractor according to the invention.
Figure 5C is a plan view of a second embodiment of the additional lifting force indicator of a fan retractor according to the invention.
Figure 6A is a cross sectional view of the preferred embodiment of the fan retractor according to the invention, taken along the line Z-Z' in figure 5A.
Figure 6B is a cross sectional view of the additional lifting force indicating window of a fan retractor according to the invention, taken along the line P-P' in figure 6A.
Figure 6C is a cross sectional view of the indicating tape channel of the fan retractor according to the invention, taken along the line Q-Q' in figure 6A.
Figure 7A is a longitudinal cross sectional view of a second leg of a fan retractor according to the invention. The second leg has an internal bore arrangement providing aspiration and/or irrigation.
Figure 7B is a longitudinal cross sectional view of a second leg of a fan retractor according to the invention. The second leg has an internal bore arrangement providing working lighting using optical fibres.
Figure 7C is a longitudinal cross sectional view of a second leg of a fan retractor according to the invention. The second leg has an internal bore arrangement providing working lighting using optical fibres brought into the second leg through a bore in the first leg.
Figure 8 illustrates a method of using a fan retractor according to the invention properitoneally to lift the abdominal wall.
Figure 8A is a longitudinal cross section of the abdomen, showing the abdominal wall, the peritoneum, and an incision through the abdominal wall as far as the peritoneum.
Figure 8B is a longitudinal cross section of the abdomen, showing the abdominal wall, the peritoneum, and the distal ends of the second legs of the fan retractor being inserted through the incision to abut the peritoneum.
Figure 8C is a longitudinal cross section of the abdomen, showing the abdominal wall, the peritoneum, and the second legs of the fan retractor being advanced between the peritoneum and the abdominal wall and progressively detaching the peritoneum from the abdominal wall.
Figure 8D is a longitudinal cross section of the abdomen, showing the abdominal wall, the peritoneum, and the fan retractor correctly positioned adjacent to the abdominal wall prior to opening the second legs of the retractor.
Figure 8E is a plan view of the abdomen showing the fan retractor correctly positioned relative to the center line of the abdomen, prior to opening the second legs of the retractor.
Figure 8F is a plan view of the abdomen showing the fan retractor symmetrically positioned relative to the center line of the abdomen after the second legs of the retractor have been opened.
Figure 8G is a longitudinal cross section of the abdomen after the abdominal wall has been lifted by the fan retractor connected to a lifting bar, showing the abdominal wall, the peritoneum, and the fan retractor with the distal parts of its second legs bent to conform with the lifted shape of the anterior abdominal wall.
Figure 9 is a cross sectional view of the abdominal wall showing the various layers of tissue.

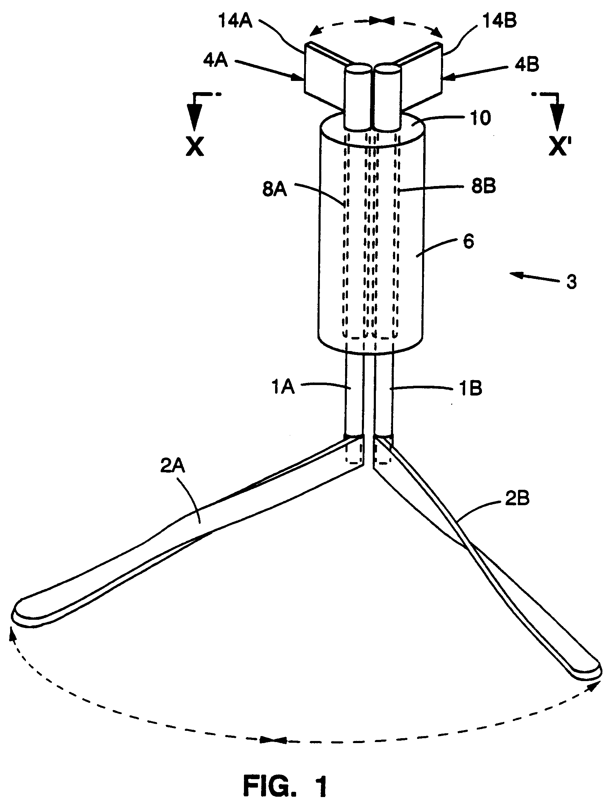

A simplified representation of a fan retractor according to the invention is shown in figure 1. The fan retractor 3 has a pair of first legs 1A and 1B, including the leg actuators 4A and 4B, a pair of second legs 2A and 2B, and a mounting block 6 to which the lifting force is applied. The second legs are shown schematically: their specific shape will be described in detail below in connection with other figures.

The mounting block 6 is cylindrical and includes two axial bores 8A and 8B, symmetrically offset from the axis, that receive the first legs 1A and 1B, respectively. The diameter of the bores is such that the bores snugly receive the first legs with the first legs free to rotate within their respective bores. The mounting block 6 is preferably moulded from a suitable plastic, such as polycarbonate, but other materials, such as stainless steel, can be used.

The first legs 1A and 1B are substantially straight, cylindrical metal rods. In the preferred embodiment, they are made from stainless steel and are about 11.4 cm (4.5") long and about 0.38 cm (0.15") in diameter.

The leg actuators 4A and 4B are attached to the end of the first legs 1A and 1B, respectively, remote from the second legs, on the opposite side of the first legs from the second legs. The leg actuators bear against the upper face 10 of the mounting block 6 and transfer the lifting force from the upper face 10 of the mounting block 6 to the first legs. The leg actuators are attached to the first legs so that they can withstand a force of several tens of kilograms exerted in the direction of the first legs.

The leg actuators 4A and 4B rotate the first legs. This changes the angular positions of the second legs 2A and 2B with respect to one another. In a fan retractor according to the invention, the second legs 2A and 2B are not capable of independent movement. If one of the second legs, for example 2A, is moved by the operating lever 14A of the leg actuator 4A through a certain angle, the other of the second legs, for example 2B, moves through substantially the same angle in the opposite direction.

In the preferred embodiment, the operating levers 14A and 14B of the leg actuators are on the opposite side of the first legs from the second legs. Moving the operating levers 14A and 14B of the actuators 4A and 4B away from one another brings the second legs towards being parallel to one another (closed position), and bringing the operating levers together splays the second legs apart (open position). This mode of operating is preferred, especially for properitoneal use, since the second legs can be opened, and the peritoneum detached from the anterior fatty layer, simply by squeezing the operating levers together. However, the operating levers may be mounted on the same side of the first legs as the second legs, if desired. Mounted in this way, the operating levers operate in the opposite sense, i.e., squeezing the operating levers together closes the second legs.

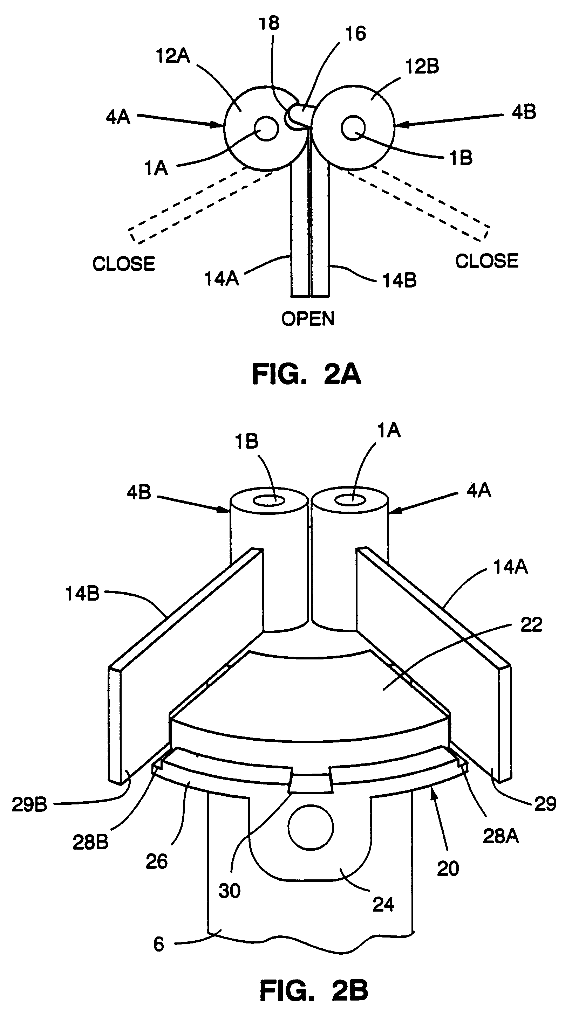

The leg actuators 4A and 4B are linked to cause the second legs to move equally and oppositely. Any rotation of one of the leg actuators causes an equal and opposite rotation in the other of the leg actuators. Many known mechanisms exist for providing such relative motion. A cross section of the leg actuators of the preferred embodiment is shown in figure 2A. The leg actuators 4A and 4B each comprise a bush 12A, 12B mounted on the respective first leg 1A, 1B, and an operating lever 14A, 14B. The operating levers 14A and 14B translate a lateral movement of the operator's thumb or finger into a rotation of the respective leg actuator 4A and 4B, and of the respective first leg 1A and 1B.

The bush 12B is provided with a peg 16 that engages with a socket 18 provided in the other bush 12A. The location of the peg and the socket can be reversed if desired. The peg and socket arrangement responds to the rotation of either one of the leg actuators 4A and 4B, and imparts an equal and opposite rotation on the other. This arrangement ensures that the second legs 2A and 2B open symmetrically and reduces the risk of one of the second legs inadvertently abutting against, and possibly penetrating, the abdominal wall. This is particularly desirable when the retractor is used properitoneally and the positions of the second legs cannot be seen directly.

Instead of the peg and socket arrangement shown, teeth can be moulded in the outer surfaces of the leg actuators 4A and 4B. Alternatively, the leg actuators can be clamped together in the direction perpendicular to the first legs 1A and 1B, and the resulting friction between them used impart the desired relative motion. Providing one or both of the leg actuators with a high friction surface is desirable in such an arrangement. As a further alternative, the rotation of the first legs themselves can be linked, instead of linking the rotation of the leg actuators.

Figure 2B shows details of the locking mechanism that holds the operating levers 14A and 14B in their open or closed positions, and hence holds the second legs 2A and 2B in their closed or open positions respectively. The sector 20 is mounted on the mounting block 6 adjacent to the operating levers 14A and 14B. In the preferred embodiment, the sector is moulded integrally with the mounting block. Relative to the operating levers 14A and 14B, the sector is slightly concave, and the operating levers 14A and 14B are biassed against the face 22 of the sector. This causes the sector to apply a frictional force to the operating levers. The frictional force holds the operating levers in any position in which they are set.

The frictional force can be released by pressing the button 24 towards the mounting block 6. The button is attached to, and preferably forms an integral part of, the sector 20. Pressing the button towards the mounting block bends the sector out of contact with the operating levers 14A and 14B, which releases the frictional force.

The skirt 26 of the sector 20 may additionally or alternatively be provided with one, two, or all of the notches 28A, 28B and 30, and the operating levers 14A and 15B provided with the operating lever extensions 29A and 29B. The notches 28A and 28B engage with the operating lever extensions 29A and 29B, respectively, when the operating levers are in the open position, i.e., when the second legs are in their closed position. The notches 28A and 28B lock the operating levers in position, and positively hold the second legs in their closed position. Because the motions of the leg actuators 4A and 4B are linked, one of the notches 28A and 28B may be omitted, if desired.

The notch 30 engages with the operating lever extensions 29A and 29B when the operating levers are in their closed position, i.e., when the second legs are in their open position. The notch 30 locks the operating levers in position, and positively holds the second legs in their open position.

If the notches 28A, 28B and 30 are provided, a clearance must be provided between the sector 20 and the operating levers 14A and 14B to enable the notches to engage with the operating lever extensions 29A and 29B. However, with this arrangement, the skirt 26 applies a frictional force against to the operating lever extensions capable of holding the operating levers, and hence the second legs, in intermediate positions.

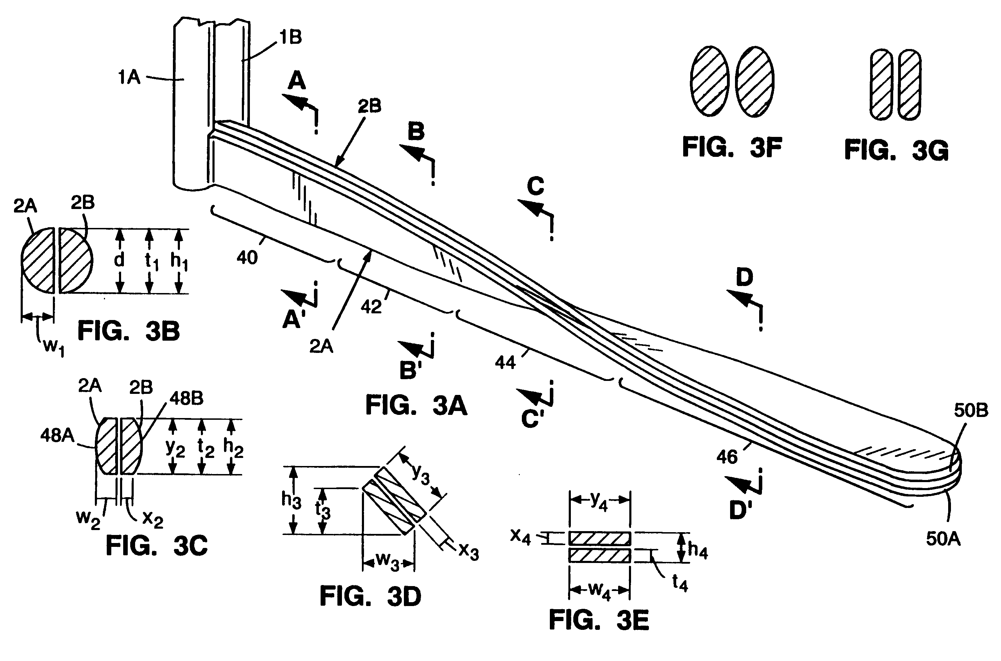

Figure 3A shows a perspective view of the second legs 2A and 2B of a fan retractor according to the invention. Figures 3B through 3E show cross sections views of the second legs at various points along their lengths. For both properitoneal use and for conventional use, the stiffness of the second legs in the lifing direction, i.e., in the direction of the first legs, is required to decrease distally from the first legs. Distally reducing the stiffness of the second legs enables the second legs to bend to conform to the shape of the raised abdomen while having sufficient strength to provide the lifting force necessary. This spreads the lifting force evenly along the length of the second legs, instead of concentrating the lifting force towards their distal ends.

For properitoneal use, second legs that are relatively flat are desirable to make it easy to insert the legs between the peritoneum and the properitoneal fatty layer. Flat legs, i.e., legs that are thin over all of their length, lack sufficient strength to exert the required lifting forces of several tens of kilograms. The inventors have discovered that second legs having an effective thickness that decreases and an effective width that increases distally from the first legs are both easy to insert under the peritoneum and are strong enough to exert the required lifting forces. The increasing thickness of the second legs towards the first legs acts as a wedge to detach the peritoneum progressively from the properitoneal fatty layer as the second legs are advanced under the peritoneum. The distally reducing thickness of the second legs also causes the stiffness of the second legs to reduce distally, which is desirable to enable the second legs to conform with the shape of the raised abdomen. The distally increasing width of the second legs helps maintain a more constant pressure against the peritoneum along the length of the second legs.

It is also desirable that the second legs form a relatively compact shape when in their closed position to reduce the size of the incision required to introduce the second legs into the abdomen, either conventionally or properitoneally.

The preferred design for the second legs that meets the requirements just stated is shown in figures 3A through 3E. Both the cross sectional area and the cross sectional shape of the second legs change distally from the first legs 1A and 1B.

The preferred second legs can be regarded as having four distinct parts. In the first part 40 of the second legs, close to the first legs, the cross section of each second leg is substantially semi-circular, as shown in figure 3B. In the first part of the second legs, the second legs have an appreciable thickness *t*₁ in the direction of the first legs. This provides considerable beam strength and stiffness, measured in the direction of the first legs, so that the required retraction force can be exerted. The semicircular cross section enables the two second legs to fit together to form a compact shape when the retractor is in its closed position

In the second part 42 of the second legs, the thickness *t*₂ of the second legs 2A and 2B remains the same as the thickness *t*₁ of the first part 40, but the width *w*₂ of the second legs progressively reduces as the cross section changes from semi-circular to rectangular. Figure 3C shows the cross section of the second legs towards the distal end of the second part where the outer surfaces 48A and 48B of the second legs are still slightly curved. At the distal end of the second part, the outer surfaces 48A and 48B are substantially straight. The length *y*₂ of the major axis of the rectangular cross section is substantially equal to the diameter *d* of the semicircular cross section of the first part 40 of the second legs, shown in figure 3B.

In the second part 42, the second legs have an appreciable thickness *t*₂ in the direction of the first legs. The beam strength and stiffness of the second legs, although appreciable, is less than in the first part 40 because the width *w*₂ of the second part is less than the width *w*₂ of the first part. The rectangular cross section enables the second parts of the second legs to fit together to form a compact shape when the retractor is in its closed position, as shown in figure 3C.

In the third part 44 of the second legs, the second legs have the same rectangular cross section as the distal part of the second part 42, but the second leg is twisted progressively through about 90 degrees over the length of the third part. The cross section of the legs about half-way along the third part, at which point the legs are twisted through about 45 degrees, is shown in figure 3D. The dimensions, *x*₃ and *y*₃ of the minor and major axes, respectively, of the rectangular cross section remain the same as the dimensions *x*₂ and *y*₂ of the minor and major axes, respectively, of the rectangular cross section in the second part. Along the third part distally from the second part, the twisting the second legs causes the effective thickness, *t*₃ of the leg to decrease and the effective width *w*₃ of the leg to increase.

Both of the second legs are twisted in the same direction through the same angle so that they will fit together to form a compact shape when the retractor is in its closed position. The two second legs are not identically twisted, however. So that the fourth part 46 of one of the second legs can fit on top of the fourth part of the other of the second legs when the retractor is in its closed position, the twist in one of the second legs is slightly offset in the direction of the first legs, and the twist in the other of the second legs is offset in the direction opposite to the direction of the first legs. Alternatively, the second legs can be made with identical twists, and be mounted on the first legs so that one forms an angle of slightly more than 90 degrees with its first leg, and the other forms an angle of slightly less than 90 degrees with its first leg.

In the third part 44, the second legs have an effective thickness *t*₃ in the direction of the first legs that progressively decreases distally from the first legs. Hence, the beam strength and stiffness of the second legs also decrease distally from the first legs. Finally, the insertion height *h*₃ of the second legs when the retractor is in its closed position progressively decreases distally from the first legs.

In the fourth part 46 of the second legs, the second legs have the same rectangular cross section as the distal part of the second part 42, but the cross section is substantially perpendicular to the cross section of the second part, as shown in figure 3E. The dimensions, *x*₄ and *y*₄ of the minor and major axes, respectively, of the rectangular cross section remain the same as the dimensions *x*₂ and *y*₂ of the minor and major axes, respectively, of the rectangular cross section in the second part.

In the fourth part 46, the second legs have an effective thickness *t*₄ in the direction of the first legs that remains substantially constant and equal to the physical thickness *y*₄ of the second legs. Hence, the beam strength and stiffness of the second legs are also substantially constant in the fourth part, and are considerably less than in the first and second parts, and in the part of the third part proximal to the first legs. The effective width *w*₄ of the second legs in the fourth part is substantially equal to the physical width *x*₄ of the legs, and is considerably greater than in the first and second parts. This considerably reduces the pressure that the fourth parts of the second legs of the retractor exert per square centimeter of abdominal wall. Finally, the insertion height *h*₄ of the fourth part of the second legs when the retractor is in its closed position is substantially equal to twice the physical thickness *y*₄ of the second legs as a result of the fourth parts of the second legs stacking on top of one another. This dimension is relative small and makes it easy to insert distal ends of the second legs of the retractor between the peritoneum and the peritoneal fat layer. Also, the distal ends 49A and 49B of the second legs are rounded to further ease insertion

In the preferred embodiment, the four parts of the second leg are approximately equal to one another in length. However, the relative lengths of the four parts can be varied to achieve a better stiffness versus distance from the first legs characteristic.

In the preferred embodiment, the first part 40 of the second legs has a semicircular cross section. An alternative oval cross section is shown in figure 3F, with the corresponding cross section of the second part 42 shown in figure 3G. The cross sections of the third and fourth parts are substantially the same as the cross sections of the third and fourth parts shown in figures 3D and 3E, respectively.

Second leg blanks having the required thickness profile for the preferred embodiment, but lacking a twisted third part 44, can be molded using a relatively simple mold. From the second leg blanks, second legs with the flat side of the first part on the left, like second leg 2A, and second legs with the flat side of the first part on the right, like second leg 2B, can be made. After a second leg blank has been moulded, it is placed in a twisting jig and supported at the distal end of its second part 42. The fourth part 46 is clamped in a rotating port of the jig. The third part of the leg is heated to soften it, and the rotating part of the jig is rotated through about 90 degrees to impart the desired twist in the third part 44 of the leg. The leg is left to cool before it is removed from the jig. The orientation of the flat side of the first part of the leg in the twisting jig determines whether a type-2A leg or a type-2B leg is made. The twisting direction is the same for both types of leg.

It is preferred, however, to make a more complex tool and to mold the second legs the required twist. With considerably more complex tools, second legs can be made having a stiffness versus distance from the first legs characteristic that is fully optimized along the length of the leg for its intended application.

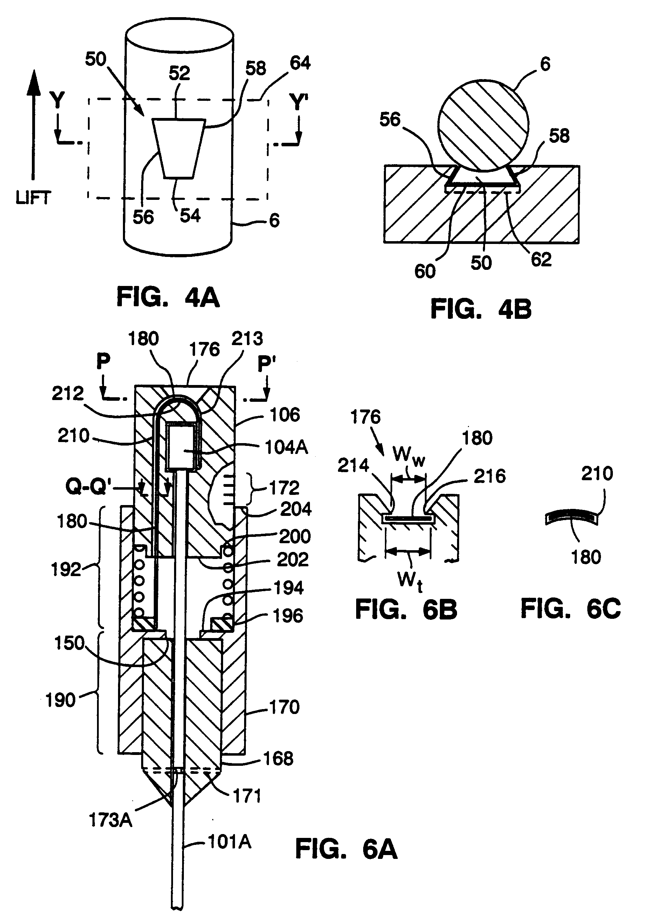

The mounting block 6 can be provided with a variety of attachments suitable for coupling to known lifting bars. In the preferred embodiment, the mounting block is provided with a dovetail connector 50, as shown in figures 4A and 4B. The dovetail connector is trapezoidal, with its parallel sides 52 and 54 perpendicular to the lifting direction, and its long parallel side 52 spaced from its short parallel side 54 in the lifting direction. The non-parallel sides, 56 and 58, form an acute angle with the front face 60. The arrangement of non-parallel sides forming an acute angle with the front face forms a positive lock with a dovetail slot 62, which is a female version of the dovetail connector 50, formed in the lifting bar adaptor 64. The lifting bar adaptor is attached to the lifting bar by conventional means (not shown).

The dovetail connector 50 and dovetail slot 62 form a unidirectional lifting force coupling. The dovetail slot and dovetail connector will transmit to the mounting block 6 a lifting force applied to the lifting bar adaptor 64 in the direction indicated by the arrow 66. A force applied in the direction opposite to that shown by the arrow 66 causes the dovetail slot to disconnect from the dovetail connector, which prevents the coupling from transmitting any force in the opposite direction.

When the retractor is used for lifting, the retractor is inserted into the abdomen and the dovetail connector 50 is engaged in the dovetail slot 62 in the lifting bar adaptor 64. The lifting bar is then raised to lift the abdominal wall, and is maintained in position during treatment. After treatment has been completed, the lifting bar is progressively lowered to return the abdominal wall to its normal position. At a point at which the force between the dovetail connector 50 and the dovetail slot 62, measured in the lifting direction, falls below zero, the dovetail connector automatically disconnects from the dovetail slot. This prevents the reverse force from being transmitted to the retractor, and indicates to the operator that the lifting bar has been lowered far enough. The dovetail connector provides a safety mechanism that prevents compression injury to the bowel if the lifting bar is lowered too far.

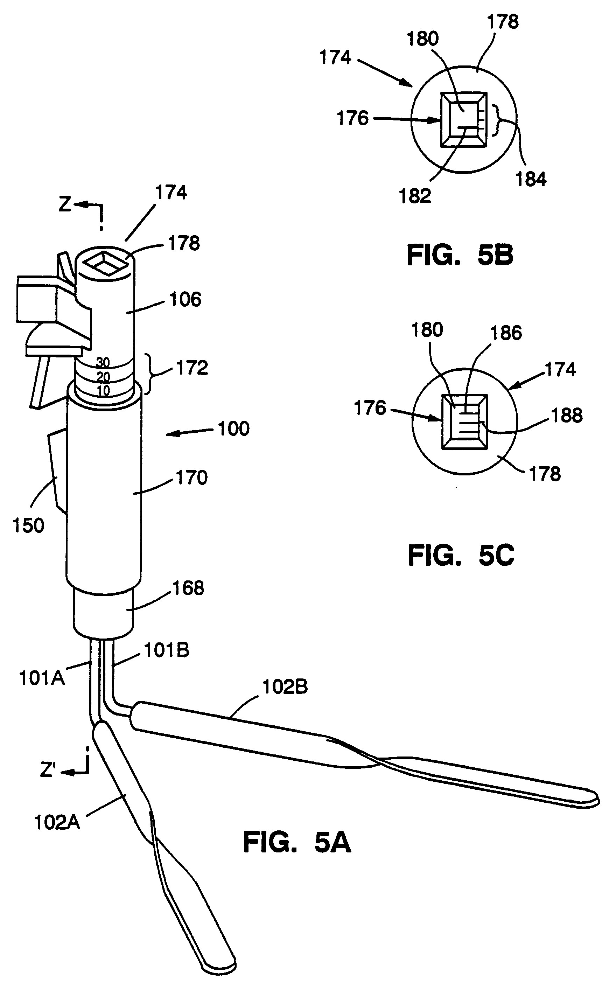

Figure 5A is a perspective view of the preferred embodiment of a fan retractor 100 including all of the aspects of the invention. In the preferred embodiment, the first legs 101A and 101B are extended by about 1" (25 mm) distally from the leg actuators 104A and 104B. The extended parts of the first legs are bent through about 90 degrees. The part of each of the first legs distal from the bend is inserted into an axial bore in the first part of the respective second leg. This arrangement is stronger than the arrangement shown in figure 1.

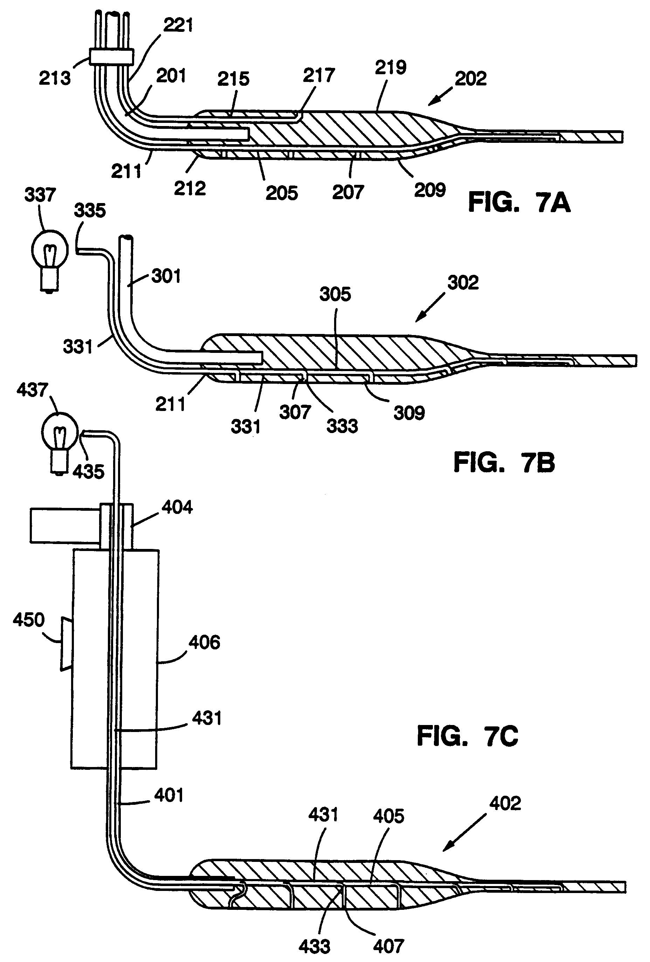

Also, in the preferred embodiment, the first legs 101A and 101B pass through offset axial bores in the cylindrical lower mounting block 168, which is slidably mounted in the lower part of the bore of the mounting sleeve 170. The main mounting block 106 is spring mounted in the upper part of the bore of the mounting sleeve 170, as will be described in connection with figure 6A below. The dovetail connector 150 is mounted on the mounting sleeve 170, instead of directly on the mounting block 168. The main mounting block can rotate relative to the mounting sleeve and the dovetail connector. This enables the second legs to face in any direction relative to the lifting bar prior to lifting. Once lifted, the lifting force prevents the main mounting block from rotating relative to the mounting sleeve, and holds the legs in the position in which they were set prior to lifting.

When the preferred embodiment of the retractor is used to retract the abdominal wall, the lifting force is applied to the mounting sleeve 170 through the dovetail connector 150. The spring mounting of the main mounting block 106 enables the relative axial positions of the mounting sleeve and main mounting block to change in response to the lifting force. The mounting sleeve moves relative to the scale 172 marked on the cylindrical surface of the mounting block, which indicates the magnitude of the lifting force. The lifting force indicator thus provided enables the lifting force to be monitored during the lifting process, and reduces the risk that an excessive lifting force will be used.

Alternatively, the lifting force scale (not shown) can be marked on the surface of the mounting sleeve 170 and a pointer (not shown) attached to the main mounting block 106 can move against the scale to indicate the lifting force.

The lifting force scale on the cylindrical surface of the mounting sleeve 170 or of the mounting block 106 is most easily seen from the side. The preferred embodiment includes an additional lifting force indicator 174 on the end face 178 of the mounting block to enable the lifting force to be monitored easily looking from above. The additional lifting force indicator is shown in detail in figures 5B and 5C. The additional lifting force indicator includes a window 176 in the end face 178 of the mounting block. A tape 180 moves in the window 176 in response to the motion of the mounting block relative to the mounting sleeve as a result of the lifting force. In the variation shown in figure 5B, the tape is marked with a reference mark 182 that moves against the scale 184 marked in the window 176, adjacent to the tape 180. In the variation shown in figure 5C, the tape is marked with the scale 186 that moves against the reference mark 188 marked in the window 176, adjacent to the tape.

The assembly of the main mounting block 106, the lower mounting block 168, and the mounting sleeve 170, and details of the additional lifting force indicator are shown in figure 6A. The mounting sleeve 170 is a tubular piece of plastic or metal having a bore. The lower part 190 of the bore receives the lower mounting block 168. A pin 171 passes through a radial bore in the lower mounting block between the first legs, and engages with a groove in each of the first legs to axially locate the lower mounting block relative to the first legs. In figure 6A, the groove 173A in the first leg 101A is shown.

The upper part 192 of the bore, which, in the preferred embodiment, has a larger diameter than the lower part 190, receives the main mounting block 106. Separating the upper and lower parts of the bore is the lip 194, which provides an end-stop for upward movement of the lower mounting block 168 in the mounting sleeve 170. The lip 194 also supports the nylon washer 196 on which the lower end of the coil spring 198 rests. The lower surface 202 of the main mounting block 106 rests on the upper end of the coil spring 198, with a circumferential groove 200 in the lower surface receiving the spring.

A lifting force applied to the dovetail connector 150 on the mounting sleeve 170 is transferred through the lip 194, the nylon washer 196, and the coil spring 198 to the upper mounting block 106, and thence to the first and second legs of the retractor. Compression of the spring 198 in response to the lifting force allows the main mounting block 106 to move relative to the mounting sleeve 170, and causes the top rim 204 of the mounting sleeve to juxtapose a different point on the scale 172, which indicates the lifting force.

The main mounting block 106 includes an upper lifting force indicator passage 210 machined or moulded into it. The passage 210 has an exit in the lower face 202 of the main mounting block, and continues distally from the lower face in a substantially straight line parallel to the curved side of the upper mounting block. Proximate to the window 176, the passage has a curved portion 212 that curves to form an arc in the window, followed by a straight portion 213 that returns at least part-way back towards the lower face 202.

The tape 180 runs through the passage 210. One end of the tape is attached to the mounting sleeve 170, preferably adjacent to the lip 194. The straight part of the passage 210 preferably has a cross section that curves across the width of the tape, as shown in figure 6C, to impart a stiffness to the tape.

The window 176 has a width *w*_{w} that is narrower than the width *w*ₜ of the tape 180, as shown in figure 6B. This enables the lips 214 and 216 to guide the tape in the curved part 212, where part of the wall of the passage is missing because of the window 176.

Compression of the spring 198 in response to the lifting force allows the main mounting block 106 to move relative to the mounting sleeve 170. This causes the tape 180 to move relative to the passage 210, and the reference mark 182 (or graduations 186) on the tape to move relative to the graduations 184 (or reference mark 188) in the window 176. The movement of the tape relative to the window indicates the lifting force.

Solid second legs are shown in figure 3A. The second legs may alternatively be provided with an arrangement of one or more internal passages, such as the arrangement of a longitudinal passage connecting to one or more transverse passages connecting to the surface of the leg shown in figures 7A-7C. An internal passage can be dedicated to a specific purpose, or can be made to receive a variety of inserts that are plugged into the internal passage to provide different capabilities, as required.

Figures 7A and 7B show two examples of internal passage arrangements in which connections are made to an internal passage at the end of the second leg proximal to the first leg. Second legs can be also made with different internal passage arrangements, including, for example, arrangements with more or fewer longitudinal passages, with more or fewer transverse passages, and/or with transverse passages connecting to the side surfaces of the second legs.

Figure 7A shows a second leg 202 attached to a first leg 201. The second leg 202 has an internal passage arrangement with two longitudinal passages 205 and 215. The longitudinal passage 205 is connected to a plurality of transverse passages, including the transverse passage 207, connecting to the lower surface 209 of the second leg. A pipe 211 is plugged into the proximal end 212 of the second leg to connect to the longitudinal passage 205. The pipe 211 is run up the outside of the first leg 201 to a suitable connection (not shown). The pipe 211 is retained in position relative to the first leg 201 by the clamp 213.

The longitudinal passage 215 is connected to the single transverse passage 217 in the upper surface 219 in the second leg. The pipe 221 connects to the longitudinal passage 215 and tuns up the outside of the first leg 201, and is retained by the clamp 213.

The internal passage arrangement can be connected to a vacuum line, which enables blood and other fluids to be aspirated from the vicinity of the fan retractor. Such an arrangement can also be used to aspirate the smoke generated by electrocautery, a process commonly used in laparoscopic procedures.

The internal passage arrangement can be connected to a suitable syringe, pump, or water line, so that fluids can be infused into the vicinity of all or parts of the second legs. The fluids can be infused into the abdomen if the fan retractor is used conventionally, or into the space between the peritoneum and the properitoneal fatty layer, if the fan retractor is used properitoneally. For example, saline can be infused for irrigation. In another example, a suitable insufflation gas can be infused for pneumoperitoneum. In procedures using local anaesthesia, a spray of anaesthetic can be sprayed into the abdomen from the fan retractor.

In the arrangement with two longitudinal passages and connecting transverse passages shown in figure 7A, the longitudinal passage 215 and transverse passage 217 can be used for infusion, and the longitudinal passage 205 and plurality of transverse passages, such as the transverse passage 207, can be used for aspiration.

In the embodiment of a fan retractor having second legs with an internal passage arrangement shown in figure 7B, the second leg 302 is attached to the first leg 301. The second leg has a longitudinal passage 305, connected to a plurality of transverse passages, including the transverse passage 307, that connect to the lower surface 309 of the second leg. A plurality of optical fibres 331 is inserted into the longitudinal passage 305 through the proximal end 211 of the second leg. Individual fibres, such as the fibre 333, or groups of fibres, are brought to the lower surface 309 through the transverse passages, such as the transverse passage 307.

The proximal end 335 of the plurality of optical fibres is connected to a suitable light source 337. Light from the light source is emitted from the ends of the fibres, such as the end of the fibre 333, in the lower surface 309 of the second legs and provides working light for the surgeon to treat the tissue being treated. The peritoneum is sufficiently translucent that light emitted from optical fibres in the second legs of the fan retractor will provide adequate working light even when the retractor is inserted properitoneally.

Instead of inserting a plurality of optical fibres into passages in the second leg, the optical fibres can be molded integrally with the second leg as part of the process of molding the second leg blanks.

Figure 7C shows an alternative arrangement in which the first leg 401 is formed using a hollow tube, the bore of which connects to the internal passage arrangement of the second leg. In the example shown, the plurality of optical fibres 431 passes through the bore of the first leg into the longitudinal passage 405, and individual fibres, such as the fibre 433, are brought out to the lower surface 409 of the second legs through a plurality of transverse passages, such as the passage 407. Otherwise, the construction of the second leg 402 is similar to the second leg 302 in figure 7B, and will not be described further.

The first leg 401 is extended through the leg actuator 404 to allow the plurality of optical fibres 431 to emerge from the bore of the first leg thus exposed. Figure 7C shows a simple form of the fan retractor that lacks a lifting force indicator (figures 5 and 6A). In fan retractors equipped with lifting force indicators (figure 6A), the additional lifting force indicator has to be relocated to provide room for the plurality of optical fibres.

Figure 7C shows an embodiment of the fan retractor having a plurality of optical fibres inserted in the internal passages of the second leg 402. Alternative versions of the embodiment of figure 7C can be made in which the internal passages are used for infusion or aspiration. In these, the infusion or aspiration pipe (similar to the pipes 211 and 221 in figure 7A) can be run inside the bore of the first leg 410. If the second leg has multiple internal passages, multiple pipes and optical fibres can be run through the bore of the first leg.

A method of using a fan retractor according to the invention properitoneally to lift the abdominal will next be described.

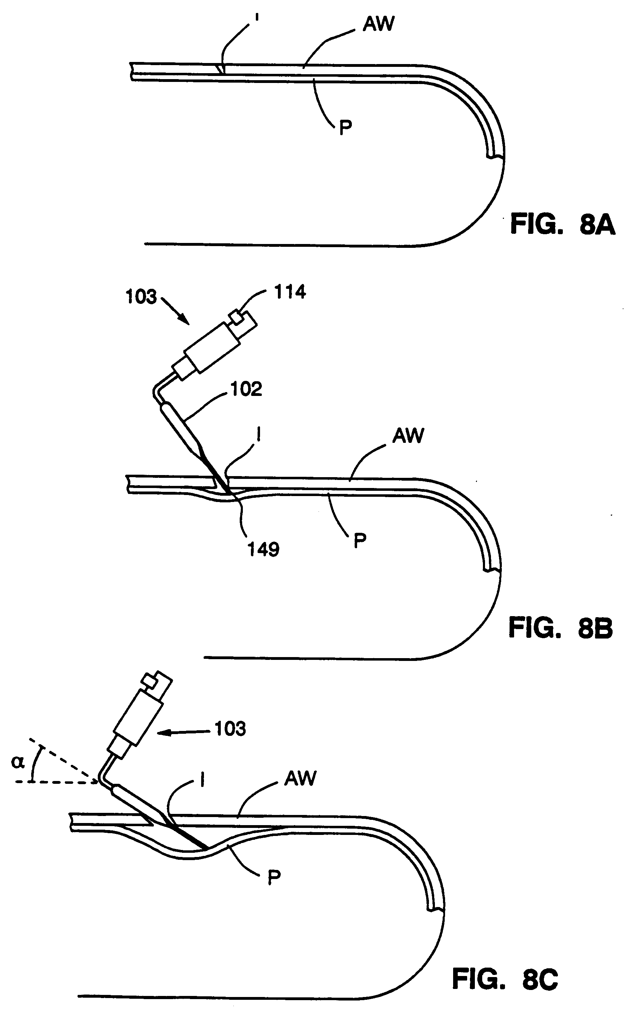

An incision I, 0.4" - 0.8" (10 - 20 mm) long is made in a suitable location in the abdominal wall AW, as shown in figure 8A. The incision is made through the skin, the subcutaneous fat, muscle and fascia, until the properitoneal fat layer is reached, just short of the peritoneum P.

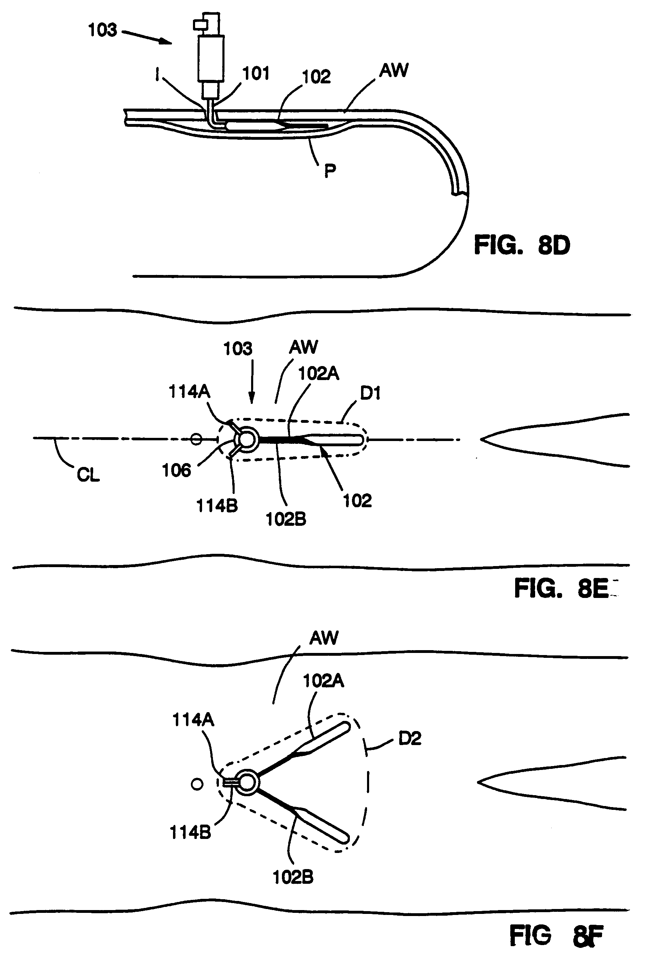

The operating levers 114 of the fan retractor 103 are manipulated to bring the second legs 102 fan retractor to their closed state if they are not in this state already. The distal ends 149 of the second legs are then inserted into the incision I and pushed through the incision until they abut against the peritoneum P. The pressure of the wide, gently curved distal ends of the second legs against the peritoneum detaches the peritoneum from the properitoneal fatty layer without piercing the peritoneum, as shown in figure 8B. The distal ends 149 of the second legs are worked into the space between the peritoneum and the properitoneal layer. The retractor is then advanced along the center line CL of the abdomen (figure 8E), keeping the angle α between the second legs and the abdominal wall as small as possible to minimize detachment of the peritoneum, as shown in figure 8C. Advancing the second legs progressively detaches the peritoneum from the properitoneal layer.

The first legs 101 abutting against the incision I limits the advancing of the second legs 102, as shown in figure 8D. The orientation of the second legs relative to the center line CL of the abdomen is checked, and the second legs are reoriented if necessary to align them along the center line (figure 8E). Since the second legs 102 have a fixed orientation relative to the main mounting block 106, orientation marks on the main mounting block can be used to indicate the direction of the second legs. The outline of the part of the peritoneum detached from the properitoneal layer is shown by the broken line D₁ in figure 8E.

The operating levers 114A and 114B are then squeezed together to force the second legs 102A and 102B apart, as shown in figure 8F. In moving apart, the second legs detach more of the peritoneum from the properitoneal layer, as shown by the dotted line D₂. While squeezing the operating levers, the orientation of the main mounting block 106 is observed to ensure that asymmetrical resistance to the opening of the second legs does not skew the symmetrical placement of the second legs relative to the center line CL. Since the second legs open symmetrically with respect to the main mounting block, any skewing of the second legs can be observed as rotation of the main mounting block. An appropriate torque applied to the main mounting block can be used to correct any skewing that occurs, and ensure that the second legs are symmetrically placed.

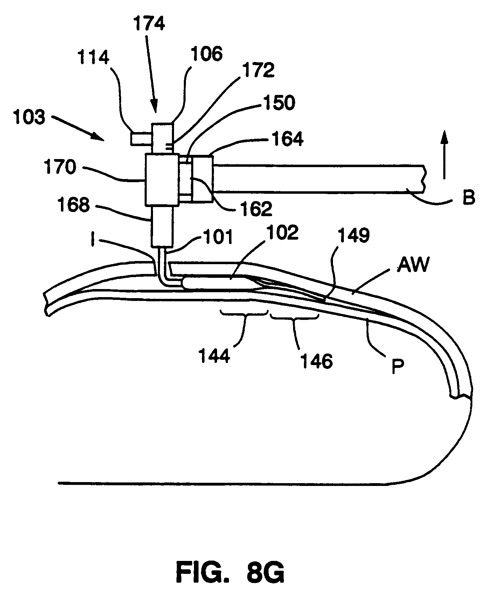

The dovetail connector 150 is coupled to the dovetail slot 162 on the lifting bar adaptor 164, as shown in figure 8G. The lifting bar adaptor is attached to the lifting bar B by conventional means (not shown). The main mounting block can rotate relative to the mounting sleeve, so the orientation of the lifting bar relative to the second legs may be changed from that shown, if desired. The lifting bar B is then raised progressively while watching one of the lifting force indicators, the scale 172 on the cylindrical wall of the main mounting block 106, or the additional lifting force indicator 174 in the end face of the main mounting block. If the lifting force comes close to the allowable limit for the procedure, the lifting force can be reduced by reducing the rate of lifting, or stopping the lifting altogether.

As the lifting force applied to the retractor increases, and the abdomen becomes more curved as a result of being lifted, the third and fourth parts 144 and 146 of the second legs bend to conform with the shape of the inside of the abdominal wall, as shown in figure 8G. The bending and the relatively large width of the distal parts of the second legs, and the symmetrical placing of the second legs within the abdomen, substantially reduce the risk of the ends 149 of the second legs traumatizing or penetrating the abdominal wall AW.

Once the required amount of lifting has been obtained, the lifting bar B is locked in position, and the treatment procedure is carried out. The incision I may be carried through the peritoneum, and used to insert endoscopes or other instruments into the abdomen. The incision I can be put to further use because the first legs 101 of the retractor occupy only a relatively small part of the incision.

If the second legs of the retractor have longitudinal passages, these may be used during the treatment procedure to provide illumination of the treatment area, and infusion and aspiration of the properitoneal area.

After treatment has been completed, the lifting bar B is progressively lowered to return the abdomen to its normal state. When the lifting force on the dovetail connector 150 falls to zero, the dovetail connector disconnects from the dovetail slot 162. This informs the operator that the lifting bar has been lowered far enough, and also prevents the lifting bar from driving the retractor into the abdomen and causing compression injury to the underlying bowel.

The operating levers 114 are moved apart to bring the second legs 102 together once more. The fan retractor 103 is then manipulated to withdraw the second legs from under the peritoneum through the incision I.

The properitoneal retraction method is preferably practiced using a fan retractor according to the invention, described above, which is specially designed for this purpose. However, the fan retractor according to the invention is not a requirement for practicing the properitoneal retraction method. The method can be practiced using other suitable retractors.

Properitoneal retraction is preferred because interposing the peritoneum between the retractor and the underlying bowel and omentum prevents the retractor from snagging the bowel or omentum. This makes the process of inserting the retractor easier, and less risky.

The fan retractor according to the invention is not limited to properitoneal use. Most of the advantages conferred by the fan retractor according to the invention can also be obtained when the fan retractor is used in the abdomen to exert its lifting force against the posterior face of the peritoneum.

The method set forth above can easily be adapted for conventional use. The incision I is carried through the peritoneum. The abdomen is preferably insufflated before the second legs of the retractor is inserted through the incision. Insufflation provides a clearance between the second legs of the retractor and the underlying bowel and omentum. A third incision is required for an endoscope to observe placement of the second legs.

When the second legs are inserted into the abdomen, care must be taken to ensure that they are kept as close to the abdominal wall as possible to reduce the chance of snagging bowel or omentum. The insertion process and the leg spreading process must be carried out under continuous observation to check for snagging. If snagging occurs, the second legs must be withdrawn, at least partially, to release the snag, and the insertion process recommenced.

Providing illumination in the vicinity of the second legs using a plurality of optical fibres inserted into internal passages in the second legs makes it easier to see snags than conventional illumination methods.

Once the second legs have been fully inserted and it is confirmed that they do not snag anything, the fan retractor is used to lift the abdominal wall, as described above.

## Claims

1. Apparatus (3) for lifting an abdominal wall, the apparatus comprising a pair of angle-shaped elements (1A + 2A; 1B + 2B) moveable relative to one another, the angle-shaped elements including:
(a) first legs (1A, 1B) disposed in a generally parallel relationship, the first legs having a direction; and
(b) second legs (2A, 2B) extending laterally from the first legs, the second legs:
(1) fanning out upon rotation of the first legs relative to one another; and
(2) being adapted for engagement with the abdominal wall;
and comprising lifting means (6) for applying a lifting force directly to the first leg of each angle-shaped element.

2. The apparatus of claim 1, **further characterized in that** the second legs (2A, 2B) are adapted for properitoneally engaging with the adominal wall and have an effective thickness (*t*) in the direction of the first legs that decreases distally from the first legs (1A, 1B).

3. The apparatus of claim 1, **further characterized in that** second legs (2A, 2B) have a stiffness in the direction of the first legs that decreases distally from the first legs (1A, 1B).

4. The apparatus of claims 1, 2, or 3, **further characterized by** a contrarotating means (4A, 4B), responsive to rotation of one of the angle-shaped elements (1A + 2A) through a first angle, for rotating the other of the first angle-shaped elements (2A + 2B) through an angle substantially equal and opposite to the first angle.

5. The apparatus of claim 4, **further characterized in that** the contrarotating means comprises a peg (12B + 16) extending radially from one of the first legs (1B) engaging in a socket (12A + 18) radially disposed in the other of the first legs (1A).

6. The apparatus of claim 4, **further characterized in that** the contrarotating means comprises a gear on the one of the first legs (1A) meshing with a gear on the other of the first legs (1B).

7. The apparatus of claim 4, **further characterized in that** the contrarotating means comprises part of one of the first legs (1A) in frictional contact with part of the other of the first legs (1B).

8. The apparatus of any of claims 1 through 7, **further characterized by** operating means (12A + 14A; 12B + 14B), attached to each angle-shaped element (1A + 1B; 2A + 2B), for rotating the angle-shaped elements with respect to one another.

9. The apparatus of claim 8, **further characterized in that** the operating means comprises a lever (14A, 14B) attached to part of each first leg (1A, 1B) distal from the second leg (2A, 2B), each lever being substantially parallel to the second legs in a first plane, and being angularly offset from the second legs in a plane perpendicular to the first plane.

10. The apparatus of claim 9, **further characterized in that:**
the angle-shaped elements (1A + 2A; 1B + 2B) have a closed position wherein the second legs are contiguous, and an open position; and
the angle-shaped elements are moved from the closed position to the open position by squeezing the levers (14A, 14B) together.

11. The apparatus of any of claims 8 through 10, **further characterized in that:**
the angle-shaped elements (1A + 2A; 1B + 2B) have a closed position wherein the second legs (2A, 2B) are contiguous, and an open position, and
the operating means additionally comprises a locking means (20), coupled to the operating means (12A + 14A + 29A; 12B + 14B + 29B), for locking the angle-shaped elements in the closed position, and for locking the angle-shaped elements in the open position.

12. The apparatus of any of claims 1 through 11, **further characterized by** a force measuring means (198) coupled to the lifting means (6), for measuring the lifting force.

13. The apparatus of claim 12, **further characterized in that**:
(a) the lifting means (6) comprises:
(1) a cylindrical sleeve (170) having a bore with a lip (194) part-way along, the bore receiving each of the first legs (101A, 101B), and
(2) a attaching means (150) for attaching the cylindrical sleeve to a lifting arm; and
(b) the force measuring means additionally comprises:
(1) a cylindrical mounting block (106), slidably mounted in the bore of the cylindrical sleeve, the mounting block including a contact face (202), and a force indicating means (172) for indicating the lifting force, the first legs (101A, 101B) each being rotatably mounted in the mounting block such that the second legs (102A, 102B) face the contact face, and
(2) a coil spring (198), disposed within the bore of the cylindrical sleeve between the lip and the contact face of the mounting block, and surrounding the first legs.

14. The apparatus of claim 13, **further characterized in that** the attaching means comprises a dovetail (150).

15. The apparatus of claim 13, **further characterized in that:**
the mounting block (106) has an axial movement relative to the cylindrical sleeve (170) in response to the lifting force; and
the force indicating means (172) indicates the lifting force in response to the axial movement of the mounting block.

16. The apparatus of any of claims 13 through 15, **further characterized in that:**
the mounting block (106) includes a surface adjacent to the cylindrical sleeve (170); and
the force indicating means comprises a scale (172) marked on the surface of the mounting block adjacent to the cylindrical sleeve.

17. The apparatus of any of claims 13 through 15, **further characterized in that:**
(a) the mounting block (106) includes:
(1) a second face (178) opposite the contact face (202), the second face including a window (176) having a reference mark (188), and
(2) a curved groove (210) adjacent to the window; and
(b) the force indicating means additionally comprises a elongate tape (180), including:
(1) an end attached to the bore of the cylindrical sleeve (170),
(2) a face marked with a scale (186), and
(3) an edge engaging in the curved groove such that the scale appears in the window adjacent to the reference mark.

18. The apparatus of any of claims 13 through 15, **further characterized in that:**
(a) the mounting block (106) includes:
(1) a second face (178) opposite the contact face (202), the second face including a window (176) marked with a scale (184), and
(2) a curved groove (210) adjacent to the window, and
(b) the indicating means additionally comprises an elongate tape (180), including:
(1) an end attached to the bore of the cylindrical sleeve (170),
(2) a face marked with a reference mark (182), and
(3) an edge engaging in the curved groove such that the reference mark appears in the window adjacent to the scale.

19. The apparatus of any of claims 1 through 18, **further characterized in that:**
the apparatus is for lifting the abdominal wall in a lifting direction; and
the lifting means (6) includes a unidirectional coupling means (150) for applying the lifting force to the lifting means in the lifting direction, and for applying a substantially zero force to the lifting means in a direction opposite to the lifting direction.

20. The apparatus of claim 19, **further characterized in that** the unidirectional coupling means comprises a dovetail connector (150).

21. The apparatus of any of claims 1 through 19, **further characterized in that** at least one of the second legs (202, 302, 402) includes an internal passage (205, 305, 405).

22. The apparatus of claim 21, **further characterized in that** at least one of the first legs (401) includes a bore connecting with the internal passage (405) in the respective second leg (402).

23. The apparatus of claim 21, **further characterized by** an optical fibre (331, 431) in the internal passage.

24. The apparatus of claim 23, **further characterized in that:**
at least one of the first legs (401) includes a bore connecting with the internal passage (405) in the respective second leg (402); and
the optical fibre (431) passes through the bore in the at least one first leg.

25. The apparatus of any of claims 21 through 24, **further characterized in that:** the second leg (202) includes a surface; and
the internal passage comprises:
a transverse passage (207, 217) connecting to the surface of the second leg, and
a longitudinal passage (205,215) connecting to the transverse passage.

26. The apparatus of any of claims 1 through 25, **further characterized in that:**
the second legs (2A, 2B) each have a cross-section and a length, the length including a first part (40) adjacent to the respective first leg (1A, 1B), a second part (42) adjacent to the first part, a third part (44) adjacent to the second part, and a fourth part (46) adjacent to the third part;
the cross-section of each of the second legs is substantially semi-circular (Fig. 3B) in the first part (40), the semi-circular cross-section having a diameter (*d*) that is substantially parallel to the direction of the first legs;
the cross-section of each of the second legs gradually changes to substantially oblong (Fig. 3C) in the second part (42), the oblong cross-section having a long axis that is substantially parallel to the first legs; and
the cross-section of each of the second legs remains substantially oblong in the third part (44), and in the fourth part (46), the oblong cross-section having a long axis that progressively changes from substantially parallel to the first legs (Fig. 3D) to substantially perpendicular to the first legs over the third part (Fig. 3E), and remains substantially perpendicular to the first legs in the fourth part to provide thickness and stiffness that decreases distally from the first legs.

27. The apparatus of any of claims 1 through 25, **further characterized in that:**
the second legs (2A, 2B) each have a cross-section and a length, the length including a first part (40) adjacent to the respective first leg (1A, 1B), a second part (42) adjacent to the first part, a third part (44) adjacent to the second part, and a fourth part (46) adjacent to the third part;
the cross-section of each of the second legs is substantially oval (Fig. 3F) in the first part (40), the substantially oval cross-section having a major axis that is substantially parallel to the direction of the first legs;
the cross-section of each of the second legs gradually changes to substantially oblong (Fig. 3F) in the second part (42), the substantially oblong cross-section having a long axis that is substantially parallel to the first legs; and
the cross-section of each of the second leges remains substantially oblong in the third part (44), and in a fourth part (46), the oblong cross-section having a long axis that progressively changes from substantially parallel to the first legs (Fig. 3D) to substantially perpendicular to the first legs (Fig. 3E) over the third part, and remains substantially perpendicular to the first legs in the fourth part to provide thickness and a stiffness that decreases distally from the first legs.

28. The apparatus of claims 26 or 27, **further characterized in that:**
the long axis of the oblong cross-section of one of the second legs (2A) changes from substantially parallel to the first legs to substantially perpendicular to the first legs in a first direction; and
the long axis of the oblong cross-section of the other of the second legs (e.g., 2B) changes from substantially parallel to the first legs to substantially perpendicular to the first legs in the first direction.

29. The apparatus of any of claims 26, 27, or 28, **further characterized in that** the cross-section of the first part (41) of the length of one of the second legs (2A) is a mirror image of the cross-section of the first part (40) of the length of the other of the second legs (2B).

30. The apparatus of any of claims 26 through 29, **further characterized in that:**
the angle-shaped elements (1A + 2A; 1B + 2B) have a closed position wherein the second legs (2A, 2B) are substantially contiguous; and, in the closed position:
the first and second parts(40, 42) of the length of one of the second legs (2A) is substantially parallel to the first and second parts (40, 42) of the length of the other of the second legs (2B) in a first plane; and
the fourth part (46) of the length of the one of the second legs (2A) is substantially parallel to the fourth part (46) of the length of the other of the second legs in a plane substantially perpendicular to the first plane.

31. The apparatus of any of claims 26 through 30, **further characterized in that** the first part (40), the second part (42), the third part (44), and the fourth part (46) of the lengths of the second legs each have a length approximately equal to one-fourth of the length of the second legs.

## Patentansprüche

1. Vorrichtung (3) zum Anheben einer Bauchdecke, wobei die Vorrichtung ein Paar von winkelförmigen Elementen (1A + 2A; 1B + 2B) umfasst, die relativ zueinander beweglich sind, und die winkelförmigen Elemente umfassen:
(a) erste Beine (1A, 1B), die in einer im allgemeinen parallelen Beziehung angeordnet sind, so dass die ersten Beine eine Richtung haben; und
(b) zweite Beine (2A, 2B), die seitlich von den ersten Beinen ausgehen, wobei die zweiten Beine:
(1) nach dem Drehen der ersten Beine relativ zueinander ausgefächert werden; und
(2) angepasst sind, um an die Bauchdecke anzuliegen;
und eine Hebeeinrichtung (6) umfasst, um eine Hebekraft direkt auf das erste Bein von jedem winkelförmigen Element anzuwenden.

2. Vorrichtung nach Anspruch 1, weiter dadurch gekennzeichnet, dass die zweiten Beine (2A, 2B) angepasst sind, um properitoneal an die Bauchdecke anzuliegen und eine wirksame Dicke (*t*) in der Richtung der ersten Beine haben, die mit zunehmender Distanz von den ersten Beinen (1A, 1B) abnimmt.

3. Vorrichtung nach Anspruch 1, weiter dadurch gekennzeichnet, dass die zweiten Beine eine Steifheit in der Richtung der ersten Beine haben, die mit zunehmender Distanz von den ersten Beinen (1A, 1B) abnimmt.

4. Vorrichtung nach Anspruch 1, 2 oder 3, weiter gekennzeichnet durch einen Gegendreheinrichtung (4A, 4B), die auf eine Drehung von einem der winkelförmigen Elemente (1A + 2A) um einen ersten Winkel durch eine Drehung des anderen der ersten winkelförmigen Elemente (2A + 2B) um einen Winkel reagiert, der im wesentlichen gleich gross wie der erste Winkel ist und diesem entgegengerichtet ist.

5. Vorrichtung nach Anspruch 4, weiter dadurch gekennzeichnet, dass die Gegendreheinrichtung einen Zapfen (12B + 16) umfasst, der radial von einem der ersten Beine (1B) ausgeht und in eine Buchse (12A + 18) eingreift, die radial im anderen der ersten Beine (1A) angeordnet ist.

6. Vorrichtung nach Anspruch 4, weiter dadurch gekennzeichnet, dass die Gegendreheinrichtung ein Zahnrad an dem einen der ersten Beine (1A) umfasst, das in ein Zahnrad am anderen der ersten Beine (1B) eingreift.

7. Vorrichtung nach Anspruch 4, weiter dadurch gekennzeichnet, dass die Gegendreheinrichtung einen Teil des einen der ersten Beine (1A) in reibschlüssigem Kontakt mit einem Teil des anderen der ersten Beine (1B) umfasst.

8. Vorrichtung nach irgend einem der Ansprüche 1 bis 7, weiter gekennzeichnet durch eine Betätigungseinrichtung (12A + 14A; 12B + 14B), die an jedem winkelförmigen Element (1A + 1B; 2A + 2B) befestigt ist, um die winkelförmigen Elemente bezüglich einander zu drehen.

9. Vorrichtung nach Anspruch 8, weiter dadurch gekennzeichnet, dass die Betätigungseinrichtung einen Hebel (14A, 14B) umfasst, der an einem Teil des ersten Beines (1A, 1B) befestigt ist, der vom zweiten Bein (2A, 2B) entfernt ist, wobei jeder Hebel in einer ersten Ebene im wesentlichen parallel zu den zweiten Beinen ist und in einer Ebene senkrecht zur ersten Ebene um einen Winkel von den zweiten Beinen verschoben ist.

10. Vorrichtung nach Anspruch 9, weiter dadurch gekennzeichnet, dass:
die winkelförmigen Elemente (1A + 2A; 1B + 2B) eine geschlossene Position haben, in welcher die zweiten Beine nebeneinander liegen, sowie eine offene Position; und
die winkelförmigen Elemente von der geschlossenen Position in die offene Position bewegt werden, indem die Hebel (14A, 14B) zusammengedrückt werden.

11. Vorrichtung nach irgend einem der Ansprüche 8 bis 10, weiter dadurch gekennzeichnet, dass:
die winkelförmigen Elemente (1A + 2A; 1B + 2B) geschlossene Positionen haben, in welchen die zweiten Beine nebeneinander liegen, sowie eine offene Position, und
die Betätigungseinrichtung zusätzlich eine Verriegelungseinrichtung (20) umfasst, die an die Betätigungseinrichtung (12A + 14A + 29A; 12B + 14B + 29B) gekoppelt ist, um die winkelförmigen Elemente in der geschlossenen Position zu verriegeln und um die winkelförmigen Elemente in der offenen Position zu verriegeln.

12. Vorrichtung nach irgend einem der Ansprüche 1 bis 11, weiter gekennzeichnet durch eine Kraftmesseinrichtung (198), die an die Hebeeinrichtung (6) gekoppelt ist, um die Hebekraft zu messen.

13. Vorrichtung nach Anspruch 12, weiter dadurch gekennzeichnet, dass:
(a) die Hebeeinrichtung (6) umfasst:
(1) eine zylindrische Muffe (170), die eine Bohrung mit einer Lippe (194) bei einer Teillänge der Bohrung hat, welche jedes der ersten Beine (101A, 101B) aufnimmt, und
(2) eine Befestigungseinrichtung (150), um die zylindrische Muffe an einem Hebearm zu befestigen; und
(b) die Kraftmesseinrichtung zusätzlich umfasst:
(1) einen zylindrischen Montageblock (106), der gleitend in der Bohrung der zylindrischen Muffe montiert ist, wobei der Montageblock eine Kontaktseite (202) und eine Kraftanzeigeeinrichtung (172) umfasst, um die Hebekraft anzuzeigen, wobei die ersten Beine (101A, 101B) beide drehbar im Montageblock montiert sind, so dass die zweiten Beine (102A, 102B) gegen die Kontaktseite zeigen, und
(2) eine Spiralfeder (198), die innerhalb der Bohrung der zylindrischen Muffe zwischen der Lippe und der Kontaktseite des Montageblockes angeordnet ist und die ersten Beine umgibt.

14. Vorrichtung nach Anspruch 13, weiter dadurch gekennzeichnet, dass die Befestigungseinrichtung einen Schwalbenschwanz (150) umfasst.

15. Vorrichtung nach Anspruch 13, weiter dadurch gekennzeichnet, dass:
der Montageblock (106) eine axiale Bewegung bezüglich der zylindrischen Muffe (170) in Abhängigkeit der Hebekraft hat; und
die Kraftanzeigeeinrichtung (172) die Hebekraft in Abhängigkeit der axialen Bewegung des Montageblockes anzeigt.

16. Vorrichtung nach irgend einem der Ansprüche 13 bis 15, weiter dadurch gekennzeichnet, dass:
der Montageblock (106) eine Oberfläche in der Nähe der zylindrischen Muffe (170) umfasst; und
die Kraftanzeigeeinrichtung (172) eine Skala umfasst, die auf der Oberfläche des Montageblockes in der Nähe der zylindrischen Muffe markiert ist.

17. Vorrichtung nach irgend einem der Ansprüche 13 bis 15, weiter dadurch gekennzeichnet, dass:
(a) der Montageblock (106) umfasst:
(1) eine zweite Seite (178) gegenüber der Kontaktseite (202), wobei die zweite Seite ein Fenster (176) umfasst, das eine Referenzmarke (188) hat, und
(2) eine gekrümmte Rille (210) in der Nähe des Fensters; und
(b) die Kraftanzeigeeinrichtung zusätzlich ein langgezogenes Band (180) einschliesst, das umfasst:
(1) ein Ende, das an der Bohrung der zylindrischen Muffe (170) befestigt ist,
(2) eine Seite, die mit einer Skala (186) markiert ist, und
(3) einen Rand, der so in die gekrümmte Rille eingreift, dass die Skala im Fenster in der Nähe der Referenzmarke erscheint.

18. Vorrichtung nach irgend einem der Ansprüche 13 bis 15, weiter dadurch gekennzeichnet, dass:
(a) der Montageblock (106) umfasst:
(1) eine zweite Seite (178) gegenüber der Kontaktseite (202), wobei die zweite Seite ein Fenster (176) umfasst, das eine Referenzmarke (184) hat, und
(2) eine gekrümmte Rille (210) in der Nähe des Fensters, und
(b) die Anzeigeeinrichtung zusätzlich ein langgezogenes Band (180) einschliesst, das umfasst:
(1) ein Ende, das an der Bohrung der zylindrischen Muffe (170) befestigt ist,
(2) eine Seite, die mit einer Referenzmarke (182) markiert ist, und
(3) einen Rand, der so in die gekrümmte Rille eingreift, dass die Referenzmarke im Fenster in der Nähe der Skala erscheint.

19. Vorrichtung nach irgend einem der Ansprüche 1 bis 18, weiter dadurch gekennzeichnet, dass:
die Vorrichtung zum Anheben der Bauchdecke in einer Heberichtung dient; und
die Hebeeinrichtung (6) eine in eine Richtung wirkende Kupplungseinrichtung (150) umfasst, um die Hebekraft in der Heberichtung auf die Hebeeinrichtung anzuwenden, und um eine Kraft von im wesentlichen null in einer Richtung entgegen der Heberichtung auf die Hebeeinrichtung anzuwenden.

20. Vorrichtung nach Anspruch 19, weiter dadurch gekennzeichnet, dass die in eine Richtung wirkende Kupplungseinrichtung einen Schwalbenschwanz-Anschluss (150) umfasst.

21. Vorrichtung nach irgend einem der Ansprüche 1 bis 19, weiter dadurch gekennzeichnet, dass mindestens eines der zweiten Beine (202, 302, 402) einen internen Durchgang (205, 305, 405) umfasst.

22. Vorrichtung nach Anspruch 21, weiter dadurch gekennzeichnet, dass mindestens eines der ersten Beine (401) eine Bohrung umfasst, die an den internen Durchgang (405) im entsprechenden zweiten Bein (402) angeschlossen ist.

23. Vorrichtung nach Anspruch 21, weiter gekennzeichnet durch eine optische Faser (331, 431) im internen Durchgang.

24. Vorrichtung nach Anspruch 23, weiter dadurch gekennzeichnet, dass:
mindestens eines der ersten Beine (401) eine Bohrung umfasst, die an den internen Durchgang (405) im entsprechenden zweiten Bein (402) angeschlossen ist; und
die optische Faser (431) durch die Bohrung in dem mindestens einen ersten Bein hindurch geht.

25. Vorrichtung nach irgend einem der Ansprüche 21 bis 24, weiter dadurch gekennzeichnet, dass:
das zweite Bein (202) eine Oberfläche umfasst; und
dei interne Durchgang umfasst:
einen Querdurchgang (207, 217), der an die Oberfläche des zweiten Beines angeschlossen ist, und
einen Längsdurchgang (205, 215), der an den Querdurchgang angeschlossen ist.

26. Vorrichtung nach irgend einem der Ansprüche 1 bis 25, weiter dadurch gekennzeichnet, dass:
die zweiten Beine (2A, 2B) je einen Querschnitt und eine Länge haben, wobei die Länge einen ersten Teil (40) neben dem entsprechenden ersten Bein (1A, 1B) umfasst, einen zweiten Teil (42) neben dem ersten Teil, einen dritten Teil (44) neben dem zweiten Teil und einen vierten Teil (46) neben dem dritten Teil;
der Querschnitt von jedem der zweiten Beine im ersten Teil (40) im wesentlichen halbkreisförmig (Fig. 3B) ist, wobei der halbkreisförmige Querschnitt einen Durchmesser (*d*) hat, der im wesentlichen parallel zur Richtung der ersten Beine ist;
der Querschnitt von jedem der zweiten Beine im zweiten Teil (42) sich im wesentlichen zu rechteckig (Fig 3C) ändert, wobei der rechteckige Querschnitt eine lange Achse hat, die im wesentlichen parallel zu den ersten Beinen ist; und
der Querschnitt von jedem der zweiten Beine im dritten Teil (44) und im vierten teil (46) im wesentlichen rechteckig bleibt, wobei der rechteckige Querschnitt eine lange Achse hat, die sich über den dritten Teil zunehmend von im wesentlichen parallel zu den ersten Beinen (Fig. 3D) zu im wesentlichen senkrecht zu den ersten Beinen (Fig. 3E) ändert, und im vierten Teil im wesentlichen senkrecht zu den ersten Beinen bleibt, um eine Dicke und Steifheit zu liefern, die mit zunehmender Distanz von den ersten Beinen abnimmt.

27. Vorrichtung nach irgend einem der Ansprüche 1 bis 25, weiter dadurch gekennzeichnet, dass:
die zweiten Beine (2A, 2B) je einen Querschnitt und eine Länge haben, wobei die Länge einen ersten Teil (40) neben dem entsprechenden ersten Bein (1A, 1B) umfasst, einen zweiten Teil (42) neben dem ersten Teil, einen dritten Teil (44) neben dem zweiten Teil und einen vierten Teil (46) neben dem dritten Teil;
der Querschnitt von jedem der zweiten Beine im ersten Teil (40) im wesentlichen oval (Fig. 3F) ist, wobei der ovale Querschnitt eine grosse Achse hat, die im wesentlichen parallel zur Richtung der ersten Beine ist;
der Querschnitt von jedem der zweiten Beine im zweiten Teil (42) sich im wesentlichen zu rechteckig (Fig 3F) ändert, wobei der rechteckige Querschnitt eine lange Achse hat, die im wesentlichen parallel zu den ersten Beinen ist; und
der Querschnitt von jedem der zweiten Beine im dritten Teil (44) und im vierten teil (46) im wesentlichen rechteckig bleibt, wobei der rechteckige Querschnitt eine lange Achse hat, die sich über den dritten Teil zunehmend von im wesentlichen parallel zu den ersten Beinen (Fig. 3D) zu im wesentlichen senkrecht zu den ersten Beinen (Fig. 3E) ändert, und im vierten Teil im wesentlichen senkrecht zu den ersten Beinen bleibt, um eine Dicke und Steifheit zu liefern, die mit zunehmender Distanz von den ersten Beinen abnimmt.

28. Vorrichtung nach Anspruch 26 oder 27, weiter dadurch gekennzeichnet, dass:
die lange Achse des rechteckigen Querschnittes von einem der zweiten Beine (2A) sich von im wesentlichen parallel zu den ersten Beinen zu im wesentlichen senkrecht zu den ersten Beinen in einer ersten Richtung ändert; und
die lange Achse des rechteckigen Querschnittes vom anderen der zweiten Beine (2B) sich von im wesentlichen parallel zu den ersten Beinen zu im wesentlichen senkrecht zu den ersten Beinen in der ersten Richtung ändert.

29. Vorrichtung nach irgend einem der Ansprüche 26, 27 oder 28, weiter dadurch gekennzeichnet, dass der Querschnitt des ersten Teils (41) der Länge von einem der zweiten Beine (2A) ein Spiegelbild des Querschnittes des ersten Teils (40) der Länge des anderen der zweiten Beine (2B) ist.

30. Vorrichtung nach irgend einem der Ansprüche 26 bis 29, weiter dadurch gekennzeichnet, dass:
die winkelförmigen Elemente (1A + 2A; 1B + 2B) eine geschlossene Position haben, bei der die zweiten Beine (2A, 2B) im wesentlichen nebeneinander liegen; und, in der geschlossenen Position:
die ersten und zweiten Teile (40, 42) der Länge von einem der zweiten Beine (2A) in einer ersten Ebene im wesentlichen parallel zu den ersten und zweiten Teilen (40, 42) der Länge des anderen der zweiten Beine (2B) sind; und
der vierte Teil (46) der Länge des einen der zweiten Beine (2A) in einer Ebene im wesentlichen senkrecht zur ersten Ebene im wesentlichen parallel zum vierten Teil (46) der Länge des anderen der zweiten Beine ist.

31. Vorrichtung nach irgend einem der Ansprüche 26 bis 30, weiter dadurch gekennzeichnet, dass der erste Teil (40), der zweite Teil (42), der dritte Teil (44) und der vierte teil (46) der Länge der zweiten Beine je eine Länge von ungefähr einem Viertel der Länge des zweiten Beines haben.

## Revendications

1. Appareil (3) pour soulever une paroi abdominale, l'appareil comprenant une paire d'éléments en forme d'angle (1A+2A; 1B+2B) mobiles l'un par rapport à l'autre, les éléments en forme d'angle comprenant:
(a) des premiers bras (1A,1B) disposés dans une relation généralement parallèle, les premiers bras ayant une direction; et
(b) des seconds bras (2A,2B) s'étendant latéralement à partir des premiers bras, les seconds bras:
(1) se déployant en éventail lors de la rotation des premiers bras l'un par rapport à l'autre; et
(2) étant adaptés pour un engagement avec la paroi abdominale;
et comprenant un moyen de soulèvement (6) pour appliquer une force de soulèvement directement au premier bras de chaque élément en forme d'angle.

2. L'appareil de la revendication 1, caractérisé en outre en ce que les seconds bras (2A,2B) sont adaptés pour un engagement pro-péritonéal avec la paroi abdominale et ont une épaisseur efficace (t) dans la direction des premiers bras qui diminue distalement à partir des premiers bras (1A,1B).

3. L'appareil de la revendication 1, caractérisé en outre en ce que les seconds bras (2A,2B) ont une rigidité dans la direction des premiers bras qui diminue distalement à partir des premiers bras (1A,1B).

4. L'appareil des revendications 1, 2 ou 3, caractérisé en outre par un moyen tournant en sens inverse (4A,4B) sensible à une rotation de l'un des éléments en forme d'angle (1A+2A) suivant un premier angle, pour tourner l'autre des premiers éléments en forme d'angle (2A+2B) d'un angle essentiellement égal et opposé au premier angle.

5. L'appareil de la revendication 4, caractérisé en outre en ce que le moyen tournant en sens inverse comprend une cheville (12B+16) s'étendant radialement à partir d'un des premiers bras (1B) engageant un manchon (12A+18) disposé radialement dans l'autre des premiers bras (1A).

6. L'appareil de la revendication 4, caractérisé en outre en ce que le moyen tournant en sens inverse comprend un engrenage sur le premier des premiers bras (1A) se mettant en prise avec un engrenage sur l'autre des premiers bras (1B).

7. L'appareil de la revendication 4, caractérisé en outre en ce que le moyen tournant en sens inverse comprend une partie de l'un des premiers bras (1A) dans un contact de friction avec une partie de l'autre des premiers bras (1B).

8. L'appareil de l'une quelconque des revendications 1 à 7, caractérisé en outre par un moyen de fonctionnement (12A+14A; 12B+14B) attaché à chaque élément en forme d'angle (1A+1B; 2A+2B) pour tourner les éléments en forme d'angle l'un par rapport à l'autre.

9. L'appareil de la revendication 8, caractérisé en outre en ce que le moyen de fonctionnement comprend un levier (14A,14B) attaché à une partie de chaque premier bras (1A,1B) distale à partir du second bras (2A,2B), chaque levier étant essentiellement parallèle aux seconds bras dans un premier plan et étant décalé angulairement par rapport aux seconds bras dans un plan perpendiculaire au premier plan.

10. L'appareil de la revendication 9, caractérisé en outre en ce que:
les éléments en forme d'angle (1A+2A; 1B+2B) ont une position fermée dans laquelle les seconds bras sont contiguës et une position ouverte; et
les éléments en forme d'angle sont déplacés à partir de la position fermée jusqu'à la position ouverte en serrant les leviers (14A,14B) ensemble.

11. L'appareil de l'une quelconque des revendications 8 à 10, caractérisé en outre en ce que:
les éléments en forme d'angle (1A+2A; 1B+2B) ont une position fermée dans laquelle les seconds bras (2A,2B) sont contiguës et une position ouverte, et
le moyen de fonctionnement comprend en outre un moyen de blocage (20) couplé au moyen de fonctionnement (12A+14A+29A; 12B+14B+29B), pour bloquer les éléments en forme d'angle dans la position fermée et pour bloquer les éléments en forme d'angle dans la position ouverte.

12. L'appareil de l'une quelconque des revendications 1 à 11, caractérisé en outre par moyen de mesure d'une force (198) couplé au moyen de soulèvement (6), pour mesurer la force de soulèvement.

13. L'appareil de la revendication 12, caractérisé en outre en ce que:
(a) le moyen de soulèvement (6) comprend:
(1) une enveloppe cylindrique (170) ayant un perçage avec une lèvre (194) à mi-chemin le long, le perçage recevant chacun des premiers bras (101A,101B) et
(2) un moyen de fixation (150) pour fixer l'enveloppe cylindrique à un bras de soulèvement; et
(b) le moyen de mesure d'une force comprend en outre:
(1) un bloc de montage cylindrique (106), monté par glissement dans le perçage de l'enveloppe cylindrique, le bloc de montage comprenant une face de contact (202), et un moyen indiquant une force (172) pour indiquer la force de soulèvement, les premiers bras (101A,101B) étant chacun montés en rotation dans le bloc de montage de sorte que les seconds bras (102A,102B) font face à la face de contact, et
(2) un ressort à boudin (198), disposé à l'intérieur du perçage de l'enveloppe cylindrique entre la lèvre et la face de contact du bloc de montage et entourant les premiers bras.

14. L'appareil de la revendication 13, caractérisé en outre en ce que le moyen de fixation comprend une queue d'aronde (150).

15. L'appareil de la revendication 13, caractérisé en outre en ce que:
le bloc de montage (106) a un mouvement axial par rapport à l'enveloppe cylindrique (170) en réponse à la force de soulèvement; et
le moyen indiquant une force (172) indique la force de soulèvement en réponse au mouvement axial du bloc de montage.

16. L'appareil de l'une quelconque des revendications 13 à 15, caractérisé en outre en ce que:
le bloc de montage (106) comprend une surface adjacente à l'enveloppe cylindrique (170) et
le moyen indiquant une force comprend une échelle (172) marquée sur la surface du bloc de montage adjacente à l'enveloppe cylindrique.

17. L'appareil de l'une quelconque des revendications 13 à 15, caractérisé en outre en ce que:
(a) le bloc de montage (106) comprend:
(1) une seconde face (178) opposée à la face de contact (202), la seconde face comprenant une fenêtre (176) ayant une marque de référence (188) et
(2) une cannelure courbe (210) adjacente à la fenêtre; et
(b) le moyen indiquant une force comprend en outre un ruban allongé (180), comprenant:
(1) une extrémité attachée au perçage de l'enveloppe cylindrique (170),
(2) une face marquée avec une échelle (186) et
(3) un bord engageant la cannelure courbe de sorte que l'échelle apparaît dans la fenêtre adjacente à la marque de référence.

18. L'appareil de l'une quelconque des revendications 13 à 15, caractérisé en outre en ce que:
(a) le bloc de montage (106) comprend:
(1) une seconde face (178) opposée à la face de contact (202), la seconde face comprenant une fenêtre (176) marquée avec une échelle (184) et
(2) une cannelure courbe (210) adjacente à la fenêtre; et
(b) le moyen indicateur comprend en outre un ruban allongé (180), comprenant:
(1) une extrémité attachée au perçage de l'enveloppe cylindrique (170),
(2) une face marquée avec une marque de référence (182) et
(3) un bord engageant la cannelure courbe de sorte que la marque de référence apparaît dans la fenêtre adjacente à l'échelle.

19. L'appareil de l'une quelconque des revendications 1 à 18, caractérisé en outre en ce que:
l'appareil sert à soulever la paroi abdominale dans une direction de soulèvement; et
le moyen de soulèvement (6) comprend un moyen de couplage unidirectionnel (150) pour appliquer la force de soulèvement au moyen de soulèvement dans la direction de soulèvement et pour appliquer une force essentiellement égale à zéro au moyen de soulèvement dans une direction opposée à la direction du soulèvement.

20. L'appareil de la revendication 19, caractérisé en outre en ce que le moyen de couplage unidirectionnel comprend un dispositif de raccordement en queue d'aronde (150).

21. L'appareil de l'une quelconque des revendications 1 à 19, caractérisé en outre en ce qu'au moins un des seconds bras (202,302,402) comprend un passage interne (205,305,405).

22. L'appareil de la revendication 21, caractérisé en outre en ce qu'au moins un des premiers bras (401) comprend un perçage se raccordant au passage interne (405) dans le second bras respectif (402).

23. L'appareil de la revendication 21, caractérisé en outre par une fibre optique (331,431) dans le passage interne.

24. L'appareil de la revendication 23, caractérisé en outre en ce que:
au moins un des premiers bras (401) comprend un perçage se raccordant au passage interne (405) dans le second bras respectif (402); et
la fibre optique (431) passe à travers le perçage au moins dans le premier des premiers bras.

25. L'appareil de l'une quelconque des revendications 21 et 24, caractérisé en outre en ce que:
le second bras (202) comprend une surface; et
le passage interne comprend:
un passage transversal (207,217) se raccordant à la surface du second bras et
un passage longitudinal (205,215) se raccordant au passage transversal.

26. L'appareil de l'une quelconque des revendications 1 à 25, caractérisé en outre en ce que:
les seconds bras (2A,2B) ont chacun une section transversale et une longueur, la longueur comprenant une première partie (40) adjacente au premier bras respectif (1A,1B), une seconde partie (42) adjacente à la première partie, une troisième partie (44) adjacente à la seconde partie et une quatrième partie (46) adjacente à la troisième partie;
la section transversale de chacun des seconds bras est essentiellement semi-circulaire (Fig. 3B) dans la première partie (40), la section transversale semi-circulaire ayant un diamètre (d) qui est essentiellement parallèle à la direction des premiers bras;
la section transversale de chacun des seconds bras change graduellement jusqu'à être essentiellement oblongue (fig. 3C) dans la seconde partie (42), la section transversale oblongue ayant un axe longitudinal qui est essentiellement parallèle aux premiers bras; et
la section transversale de chacun des seconds bras reste essentiellement oblongue dans la troisième partie (44), et dans la quatrième partie (46), la section transversale oblongue ayant un axe longitudinal qui change progressivement depuis essentiellement parallèle aux premiers bras (fig. 3D) jusqu'à essentiellement perpendiculaire aux premiers bras sur la troisième partie (fig. 3E), et reste essentiellement perpendiculaire aux premiers bras dans la quatrième partie pour fournir une épaisseur et une rigidité qui diminuent distalement à partir des premiers bras.

27. L'appareil de l'une quelconque des revendications 1 à 25, caractérisé en outre en ce que:
les seconds bras (2A,2B) ont chacun une section transversale et une longueur, la longueur comprenant une première partie (40) adjacente au premier bras respectif (1A,1B), une seconde partie (42) adjacente à la première partie, une troisième partie (44) adjacente à la seconde partie et une quatrième partie (46) adjacente à la troisième partie;
la section transversale de chacun des seconds bras est essentiellement ovale (Fig. 3F) dans la première partie (40), la section transversale essentiellement ovale ayant un axe principal qui est essentiellement parallèle à la direction des premiers bras;
la section transversale de chacun des seconds bras change graduellement jusqu'à être essentiellement oblongue (fig. 3F) dans la seconde partie (42), la section transversale essentiellement oblongue ayant un axe longitudinal qui est essentiellement parallèle aux premiers bras; et
la section transversale de chacun des seconds bras reste essentiellement oblongue dans la troisième partie (44), et dans une quatrième partie (46), la section transversale oblongue ayant un axe longitudinal qui change progressivement depuis essentiellement parallèle aux premiers bras (fig. 3D) jusqu'à essentiellement perpendiculaire aux premiers bras (fig. 3E) sur la troisième partie, et reste essentiellement perpendiculaire aux premiers bras dans la quatrième partie pour fournir une épaisseur et une rigidité qui diminuent distalement à partir des premiers bras.

28. L'appareil des revendications 26 ou 27, caractérisé en outre en ce que:
l'axe longitudinal de la section transversale oblongue de l'un des seconds bras (2A) change depuis essentiellement parallèle aux premiers bras jusqu'à essentiellement perpendiculaire aux premiers bras dans une première direction; et
l'axe longitudinal de la section transversale oblongue de l'autre des seconds bras (p.ex. 2B) change depuis essentiellement parallèle aux premiers bras jusqu'à essentiellement perpendiculaire aux premiers bras dans la première direction.

29. L'appareil de l'une quelconque des revendications 26, 27 ou 28, caractérisé en outre en ce que la section transversale de la première partie (41) de la longueur de l'un des seconds bras (2A) est une image miroir de la section transversale de la première partie (40) de la longueur de l'autre des seconds bras (2B).

30. L'appareil de l'une quelconque des revendications 26 à 29, caractérisé en outre en ce que:
les éléments en forme d'angle (1A+2A; 1B+2B) ont une position fermée dans laquelle les seconds bras (2A,2B) sont essentiellement contiguës; et, dans la position fermée:
les premières et secondes parties (40,42) de la longueur de l'un des seconds bras (2A) sont essentiellement parallèles aux première et seconde parties (40,42) de la longueur de l'autre des seconds bras (2B) dans un premier plan; et
la quatrième partie (46) de la longueur de l'un des seconds bras (2A) est essentiellement parallèle à la quatrième partie (46) de la longueur de l'autre des seconds bras dans un plan essentiellement perpendiculaire au premier plan.

31. L'appareil de l'une quelconque des revendications 26 à 30, caractérisé en outre en ce que la première partie (40), la seconde partie (42), la troisième partie (44) et la quatrième partie (46) des longueurs des seconds bras ont chacune une longueur approximativement égale à un quart de la longueur des seconds bras.
